Europäisches Patentamt

European Patent Office

Office européen des brevets

(11) Veröffentlichungsnummer: **0 307 938**
**A1**

(12) **EUROPÄISCHE PATENTANMELDUNG**

(21) Anmeldenummer: 88115201.1

(22) Anmeldetag: 16.09.88

(51) Int. Cl.⁴: **A61F 7/10**

(30) Priorität: 17.09.87 DE 8712586 U

(43) Veröffentlichungstag der Anmeldung:
22.03.89 Patentblatt 89/12

(84) Benannte Vertragsstaaten:
AT BE CH DE ES FR GB GR IT LI LU NL SE

(71) Anmelder: HEWI-PACK Verpackungs-Service
GmbH
Gerresheimer Landstrasse 119
D-4000 Düsseldorf 12(DE)

(72) Erfinder: Hecht, Michael
Gerresheimer Landstrasse 119
D-4000 Düsseldorf 12(DE)

(74) Vertreter: Kilian, Helmut, Dr. et al
Wilhelms, Kilian & Partner Patentanwälte
Eduard-Schmid-Strasse 2
D-8000 München 90(DE)

(54) Kältepackung.

(57) Bei einer Packung zur Applikation von Kälte am menschlichen oder tierischen Körper mit mindestens zwei Applikationsflächen, von denen eine eine glatte Oberfläche aufweist, ist vorgesehen, daß eine zweite der Applikationsflächen eine textil- oder vliesartige Oberfläche aufweist.

Fig. 1

EP 0 307 938 A1

# Kältepackung

Die Erfindung betrifft eine Packung zur Applikation von Kälte am menschlichen oder tierischen Körper, mit mindestens zwei Applikationsflächen, von denen eine eine glatte Oberfläche aufweist.

Es sind Kältepackungen bekannt, bei welchen das Kältemittel zwischen zwei Kunststoffbahnen mit typischer glatter Oberfläche eingeschweißt ist. Sobald das Kältemittel seine Kälte entwickelt, beschlagen die Oberflächen der Packung mit einem Feuchtigkeitsfilm aus der Umgebungsluft, so daß sich mit derartigen Packungen besonders gut Gleitbewegungen auf der behandelten Körperoberfläche ausführen lassen, wie man sie sonst bei der Kühlung mit Eisstücken vornimmt. Das sich bei der Behandlung einstellende Gefühl ist das Gefühl ausgeprägter nasser Kälte.

Ferner bekannt sind Kältepackungen, bei denen das Kältemittel zwischen Faservliesen eingeschweißt ist. Die dadurch erreichte stumpfe, textilartig erscheinende Oberfläche verhindert, daß Feuchtigkeit zu einem Flüssigkeitsfilm kondensiert, so daß dann zwar Gleitbewegungen auf den zu behandelnden Körperstellen nicht mehr gut ausführbar sind, dafur das Kältegefühl aber trocken und weniger ausgeprägt ist.

Aufgabe der Erfindung ist es, eine Kältepackung vorzuschlagen, deren Anwendungsbereich breiter als der von bekannten ist.

Diese Aufgabe wird erfindungsgemäß durch eine Kältepackung gemäß dem Schutzanspruch gelöst.

Im folgenden wird eine Ausführungsform der Erfindung anhand der beigefügten Zeichnung beschrieben. Auf dieser zeigt

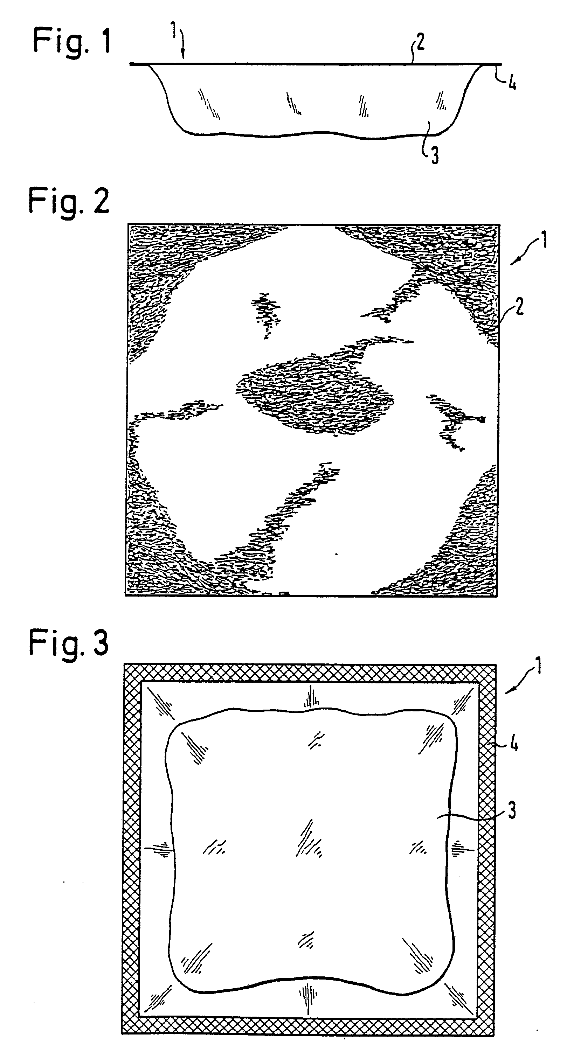

Fig. 1 eine Seitenansicht einer Kältepackung,

Fig. 2 eine Ansicht der Kältepackung von oben, und

Fig. 3 eine Ansicht der Kältepackung von unten.

Die insgesamt mit 1 bezeichnete Kältepackung weist zwischen zwei Kunststoffbahnen 2 und 3, die langs einer Schweißnaht 4 miteinander verschweißt sind, ein Kältemittel, beispielsweise Ammoniumnitratgranulat, und Wasser auf, wobei sich das Wasser zunächst in einem miteingeschweißten verschlossenen Beutel befindet, der für den Einsatz der Kältepackung mit der Hand zerquetscht wird, wonach das Ammoniumnitrat mit dem Wasser in Berührung kommt und dabei in Lösung geht, was zu einer Abkühlung führt.

Die eine der beiden Bahnen, 2, ist ein Kunststoffvlies, also eine Bahn mit rauher stoff- bzw. textilartiger Oberfläche. Auch eine textil- oder vliesartig beschichtete glatte Kunststoffolie kommt dabei in Betracht.

Die andere Bahn, 3, die im vorliegenden Fall ausgetieft ausgebildet ist, während die vliesartige Bahn als plane Deckbahn vorliegt, besteht aus einem Kunststoff mit glatter Oberfläche ohne die Saugwirkung, wie sie eine vliesartige Oberfläche entwickelt, so daß sich auf dieser Bahn beim Abkühlen ein Feuchtigkeits- bzw. Wasserfilm aus aus der Luft kondensiertem Wasser bildet.

Im Einsatz vermittelt diese glatte Bahn 3 ein Gefühl nasser Kälte wie bei einer Behandlung mit einem Eisblock, während die Bahn 2 vliesartiger bzw. textilartiger Oberfläche ein Gefühl trockener Kälte vermittelt, wobei das subjektive Kältegefühl, bei objektiv gleicher Temperatur, weniger stark wie bei der glatten Bahn 3 ist.

Die beschriebene Kältepackung hat damit ein breites Anwendungsfeld, das über dasjenige von Packungen mit nur glatten bzw. von Packungen mit nur stoffartigen Oberflächen hinausgeht.

## Ansprüche

Packung zur Applikation von Kälte am menschlichen oder tierischen Körper mit mindestens zwei Applikationsflächen, von denen eine eine glatte Oberfläche aufweist, dadurch **gekennzeichnet,** daß eine zweite der Applikationsflächen eine textil- oder vliesartige Oberfläche aufweist.

Fig. 1

Fig. 2

Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A- 147 233 (COTTON) <br> * Seite 1, Zeilen 39-45,64-74 * <br> --- | 1 | A 61 F 7/10 |
| X | GB-A- 191 643 (SAMPSON) <br> * Seite 2, Zeilen 13-18; Figuren 9,10 * <br> --- | 1 | |
| A | FR-A- 456 177 (HOSTETTER) <br> * Seite 1, Zeilen 43-48; Figuren 1,2 * <br> --- | 1 | |
| A | DE-U-8 504 306 (WEISE-RICHTER) <br> * Anspruch * <br> --- | 1 | |
| A | US-A-4 114 620 (MOORE et al.) <br> * Spalte 3, Zeilen 33-42 * <br> ----- | 1 | |

RECHERCHIERTE SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 16-12-1988 | GLAS J. |